# EUROPEAN PATENT SPECIFICATION

(11) **EP 1 545 679 B1**
(45) Date of publication and mention of the grant of the patent: **17.02.2010**
(21) Application number: 03759489.2
(22) Date of filing: 25.09.2003
(51) Int. Cl.: A61M 25/01

(54) **RADIOPAQUE NITINOL ALLOYS FOR MEDICAL DEVICES**
STRAHLUNGSUNDURCHLÄSSIGE NITINOLLEGIERUNGEN FÜR MEDIZINISCHE GERÄTE
ALLIAGES DE NITINOL RADIO-OPAQUES POUR DISPOSITIFS MEDICAUX

(30) Priority: 04.10.2002 US 264619
(43) Date of publication of application: 29.06.2005
(73) Proprietor: Abbott Cardiovascular Systems Inc., Santa Clara, CA 95054 (US)
(72) Inventor: BOYLAN, John, F., Murrieta, CA 92562 (US); COX, Daniel, L., Palo Alto, CA 94301 (US)
(74) Representative: McLeish, Nicholas Alistair Maxwell
(86) International application number: PCT/US2003/030111
(87) International publication number: WO 2004/033016

(56) References cited:
- WO-A-97/24978
- US-A1- 2001 049 549
- US-B1- 6 329 069
- US-B1- 6 375 628

## Description

The present invention generally relates to intracorporeal and self-expanding medical devices. More precisely, the present invention relates to guide wires.

A guide wire according to the preamble of claim 1 is described for example US-A-6329069.

Stents are typically implanted in a body lumen, such as carotid arteries, coronary arteries, peripheral arteries, veins, or other vessels to maintain the patency of the lumen. These devices are frequently used in the treatment of atherosclerotic stenosis in blood vessels especially after percutaneous transluminal angioplasty (PTA) or percutaneous transluminal coronary angioplasty (PTCA) procedures with the intent to reduce the likelihood of restenosis of a vessel. Stents are also used to support a body lumen, tack-up a flap or dissection in a vessel, or in general where the lumen is weak to add support.

During PTCA procedures it is common to use a dilatation catheter to expand a diseased area to open the patient's lumen so that blood flows freely. Despite the beneficial aspects of PTCA procedures and its widespread and accepted use, it has several drawbacks, including the possible development of restenosis and perhaps acute thrombosis and sub-acute closure. This recurrent stenosis has been estimated to occur in seventeen to fifty percent of patients despite the initial PTCA procedure being successful. Restenosis is a complex and not fully understood biological response to injury of a vessel which results in chronic hyperplasia of the neointima. This neointimal hyperplasia is activated by growth factors which are released in response to injury. Acute thrombosis is also a result of vascular injury and requires systemic antithrombotic drugs and possibly thrombolytics as well. This therapy can increase bleeding complications at the catheter insertion site and may result in a longer hospital stay. Sub-acute closure is a result of thrombosis, elastic recoil, and/or vessel dissection.

Several procedures have been developed to combat restenosis and sub-acute or abrupt closure, one of which is the delivery and implanting of an intravascular stent. Stents are widely used throughout the United States and in Europe and other countries. Generally speaking, the stents can take numerous forms. One of the most common is a generally cylindrical, hollow tube that holds open the vascular wall at the area that has been dilated by a dilation catheter. One highly regarded stent used and sold in the United States is known under the tradename ACS Multi-Link Stent, which is made by Advanced Cardiovascular Systems, Inc., Santa Clara, California.

In expandable stents that are delivered with expandable catheters, such as balloon catheters, the stents are positioned over the balloon portion of the catheter and are expanded from a reduced diameter to an enlarged diameter greater than or equal to the inner diameter of the arterial wall by inflating the balloon. Stents of this type can be expanded to an enlarged diameter by deforming the stent, by engagement of the stent walls with respect to one another, and by one way engagement of the stent walls together with endothelial growth onto and over the stent.

Examples of intravascular stents can be found in U.S. Patent No. 5,292,331 (Boneau); U.S. Patent No. 4,580,568 (Gianturco); U.S. Patent No. 4,856,516 (Hillstead); U.S. Patent No. 5,092,877 (Pinchuk); and U.S. Patent No. 5,514,154 (Lau et al.).

The problem with some prior art stents, especially those of the balloon expandable type, is that they are often stiff and inflexible. These balloon expandable type stents are commonly formed from stainless steel alloys and the stents are constructed so that they are expanded beyond their elastic limit. As a result, such stents are permanently deformed by the inflation balloon beyond their elastic limits to hold open a body lumen and thus maintain patency of that body lumen. There are several commercially available balloon expandable stents that are widely used; they are generally implanted in the coronary arteries after a PTCA procedure mentioned earlier.

Stents are often times implanted in vessels that are closer to the surface of the body, such as in the carotid arteries in the neck or in peripheral arteries and veins in the leg. Because these stents are so close to the surface of the body, they are particularly vulnerable to impact forces that can partially or completely collapse the stent and thereby block fluid flow in the vessel. Other forces can impact balloon expandable stents and cause similar partial or total vessel blockage. For instance, under certain conditions, muscle contractions might also cause balloon expandable stents to collapse partially or completely. The collapse occludes the lumen and restricts blood flow in the vessel in which they are implanted.

Since balloon expandable stents are plastically deformed, once collapsed or crushed they remain so, permanently blocking the vessel. Thus, balloon expandable stents under certain conditions might pose an undesirable condition for the patient.

Self-expanding stents as the name implies self-expand through the properties of the material constituting the stent. The inflation force of a balloon catheter is usually not necessary to deploy this kind of stent.

Important applications including those mentioned above have prompted designers to seek out superelastic shape memory alloys to exploit the materials' properties in their self-expanding stents. Examples of applying superelastic nickel-titanium alloys to a self-expanding stent and other medical devices are disclosed in U.S. Patent Nos. 4,665,906; 5,067,957; 5,190,546; and 5,597,378 to Jervis and U.S. Patent No. 4,503,569 to Dotter. Another example is disclosed in European Patent Application Publication No. EP0873734A2, entitled "Shape Memory Alloy Stent." This publication suggests a stent for use in a lumen in a human or animal body having a generally tubular body formed from a shape memory alloy which has been treated so that it exhibits enhanced elastic properties. The publication further suggests use of specified ternary elements in a nickel-titanium alloy to obtain desired engineering characteristics.

In a typical PTCA procedure, a guiding catheter having a preformed distal tip is percutaneously introduced into the cardiovascular system of a patient in a conventional Seldinger technique and advanced therein until the distal tip is seated in the ostium of a desired coronary artery. A guide wire is positioned within an inner lumen of a dilatation catheter and then both are advanced through the guiding catheter to the distal end thereof. The guide wire is advanced out of the distal end of the guiding catheter into the patient's coronary vasculature until the distal end of the guide wire crosses a lesion to be dilated, then the dilatation catheter having an inflatable balloon on the distal portion thereof is advanced into the patient's coronary anatomy over the previously introduced guide wire until the balloon of the dilatation catheter is properly positioned across the lesion. Once in position across the lesion, the balloon is inflated to compress the arteriosclerotic plaque of the lesion against the inside of the artery wall. Through various other known procedures, a stent may be deployed and implanted at the lesion.

Conventional guide wires for angioplasty and other vascular procedures usually comprise an elongated core member with one or more tapered sections near the distal end and a flexible body such as a helical coil disposed about the distal portion of a core member. A shapeable member, which may be the distal extremity of the core member or a separate shaping ribbon that is secured to the distal extremity of the core member, extends through the flexible body and is secured to a rounded plug at the distal end of the flexible body. A torque applying mechanism is provided on the proximal end of the core member to rotate, and thereby steer, the guide wire while it is being advanced through the patient's vascular system.

A major requirement for guide wires and other guiding members, whether they be solid wire or tubular members, is that they have sufficient column strength to be pushed through a patient's vascular system or other body lumen without kinking. However, they must also be flexible enough to avoid damaging the blood vessel or other body lumen through which they are advanced. Efforts have been made to improve both the strength and flexibility of guide wires to make them more suitable for their intended uses, but these two properties are for the most part diametrically opposed to one another in that an increase in one usually involves a decrease in the other.

The prior art makes reference to the use of alloys such as nitinol (NiTialloy) which have shape memory and/or superelastic characteristics in medical devices that are designed to be inserted into a patient's body. Because of these properties, nitinol has been employed in the fabrication of guide wires, stents, and the like.

Use of a ternary element in a superelastic stent is also shown in, for example, U.S. Patent No. 5,907,893 to Zadno-Azizi et al. As a general proposition, there have been attempts at adding a ternary element to nickel-titanium alloys as disclosed in, for instance, U.S. Patent No. 5,885,381 to Mitose et al.

Clearly, self-expanding, nickel-titanium stents are useful and valuable to the medical field. But a distinct disadvantage with self-expanding nickel-titanium stents is the fact that they are not sufficiently radiopaque as compared to a comparable structure made from gold or tantalum. For example, radiopacity permits the cardiologist or physician to visualize the procedure involving the stent through use of fluoroscopes or similar radiological equipment. Good radiopacity is therefore a useful feature for self-expanding nickel-titanium stents and guide wires to have.

Radiopacity can be improved by increasing the strut thickness of the nickel-titanium stent. But increasing strut thickness detrimentally affects the flexibility of the stent, which is a quality necessary for ease of delivery. Another complication is that radiopacity and radial force co-vary with strut thickness. Also, nickel-titanium is difficult to machine and thick struts exacerbates the problem.

Radiopacity can be improved through coating processes such as sputtering, plating, or co-drawing gold or similar heavy metals onto the stent. These processes, however, create complications such as material compatibility, galvanic corrosion, high manufacturing cost, coating adhesion or delamination, biocompatibility, loss of coating integrity following collapse and deployment of the stent, etc.

Radiopacity can also be improved by alloy addition. One specific approach is to alloy the nickel-titanium with a ternary element. What has been needed and heretofore unavailable in the prior art is a superelastic nickel-titanium stent that includes a ternary element to increase radiopacity yet preserves the superelastic qualities of the nitinol.

### SUMMARY OF THE INVENTION

An example which is not comprised within the scope of the appended claims relates to a radiopaque medical device, such as a stent, for use or implantation in a body lumen. In a preferred embodiment, a radiopaque medical device, such as a stent, is constructed from a tubular-shaped body having a thin wall defining a strut pattern; wherein the tubular body includes a superelastic, nickel-titanium alloy, and the alloy further includes a ternary element selected from the group of chemical elements consisting of iridium, platinum, gold, rhenium, tungsten, palladium, rhodium, tantalum, silver, ruthenium, or hafnium. In a preferred embodiment, the stent has 42.8 atomic percent nickel, 49.7 atomic percent titanium, and 7.5 atomic percent platinum.

As a result, the stent is highly radiopaque as compared to an identical structure made of medical grade stainless steel that is coated with a thin layer of gold. From another perspective, for a given stent having a certain level of radiopacity, the present invention stent having identical dimensions and strut pattern has at least a 10 percent reduction in strut thickness yet maintains that same level of radiopacity.

Self-expanding nitinol stents are collapsed (that is, loaded) and then constrained within a delivery system. At the point of delivery, the stent is released (that is, unloaded) and allowed to return to its original diameter. The stent is designed to perform various mechanical functions within the lumen, all of which are based upon the lower unloading plateau stress. Therefore, it is crucial that the ternary element alloyed with the binary nickel-titanium does not diminish the superelastic characteristics of the nickel-titanium.

To achieve the sufficient degree of radiopacity yet maintaining the superelastic engineering properties of a binary nickel-titanium, preferably, the radiopaque stent of the present invention includes platinum whose atomic percent is greater than or equal to 2.5 and less than or equal to 15. In an alternative embodiment, the nickel-titanium is alloyed with palladium whose atomic percent is greater than or equal to 2.5 and less than or equal to 20. With such compositions, the stress-strain hysteresis curve of the present invention radiopaque nitinol alloy closely approximates the idealized stress-strain hysteresis curve of binary nickel-titanium.

The present example further contemplates a method for providing a radiopaque nitinol stent. In a preferred embodiment, the method entails providing a tubular-shaped body having a thin wall, wherein the body includes a superelastic nickel-titanium alloy and the alloy further includes a ternary element selected from the group of chemical elements consisting of iridium, platinum, gold, rhenium, tungsten, palladium, rhodium, tantalum, silver, ruthenium, or hafnium; forming a strut pattern; wherein the stent is highly radiopaque. The step of providing a tubular-shaped body includes melting nickel, titanium, and the ternary element and cooling the mixture to form an alloy ingot, hot forming the alloy ingot, hot or cold forming the alloy ingot into a cylinder, drilling the cylinder to form tubing, cold drawing the tubing, and annealing the tubing.

The present invention of course envisions the minor addition of a quaternary element, for example, iron, to further enhance the alloy's formability or its thermomechanical properties. In short, the presence of elements in addition to the ternary elements cited above is contemplated.

In a preferred embodiment, an austenite finish temperature (Af) of the superelastic alloy in the stent is greater than or equal to zero and less than or equal to 37 degrees C. Also in the preferred embodiment, the ingot after melting includes an austenite finish temperature (Af) of greater than or equal to 0 degrees C and less than or equal to 40 degrees C. The tubing includes an austenite finish temperature (Af) of greater than or equal to -15 degrees C and less than or equal to 15 degrees C.

The present invention is applied to a guide wire comprising an elongate core having proximal and distal core sections, wherein the distal core section includes a superelastic alloy, and the alloy further includes a ternary element selected from the group of chemical elements consisting of iridium, platinum, gold, rhenium, tungsten, palladium, rhodium, tantalum, silver, ruthenium, or hafnium; and wherein the distal core section is substantially radiopaque.

Other features and advantages of the present invention will become more apparent from the following detailed description of the invention when taken in conjunction with the accompanying exemplary drawings.

### BRIEF DESCRIPTION OF THE DRAWINGS

FIGURE 1 is a side elevational view, partially in section, depicting a stent (not comprised within the scope of the appended claims) mounted on a delivery catheter and expanded within a damaged vessel, pressing a damaged vessel lining against the vessel wall.
FIG. 2 is a side elevational view partially in section, depicting an expanded stent (not comprised within the scope of the appended claims) within the vessel after withdrawal of the delivery catheter.
FIG. 3 is an idealized stress-strain hysteresis curve for a superelastic material.
FIG. 4 is a plan view of the flattened strut pattern of an exemplary embodiment superelastic stent (not comprised within the scope of the appended claims).
FIG. 5 is a group of empirical data curves illustrating the highly similar stress-strain relationships among binary nitinol and the nickel-titanium-palladium and nickel-titanium-platinum alloys used in the present invention.
FIG. 6 is a side elevational view of the guide wire embodying features of the present invention.
FIG. 7 is a side elevational view partially in section of an alternative embodiment guide wire having features of the present invention.
FIG. 8 is a cross-sectional view taken along line 8-8 of the guide wire shown in FIG 7.
FIG. 9 is a cross-sectional view taken along line 9-9 of the guide wire shown in FIG 7.
FIG. 10 is a side elevational view of an alternative embodiment guide wire having features of the present invention and employing a shaping ribbon.

### DETAILED DESCRIPTION OF THE PREFERRED EMBODIMENTS

The present invention relates to a medical device made of radiopaque nitinol. For the sake of illustration, the following exemplary embodiments are directed to guide wires and stents, although it is understood that the present invention is applicable to other medical devices usable in a body lumen as well.

The stents can have virtually any configuration that is compatible with the body lumen in which they are implanted. The stent should preferably be configured so that there is a substantial amount of open area and preferably the open area to metal ratio is at least 80 percent. The stent should also be configured so that dissections or flaps in the body lumen wall are covered and tacked up by the stent.

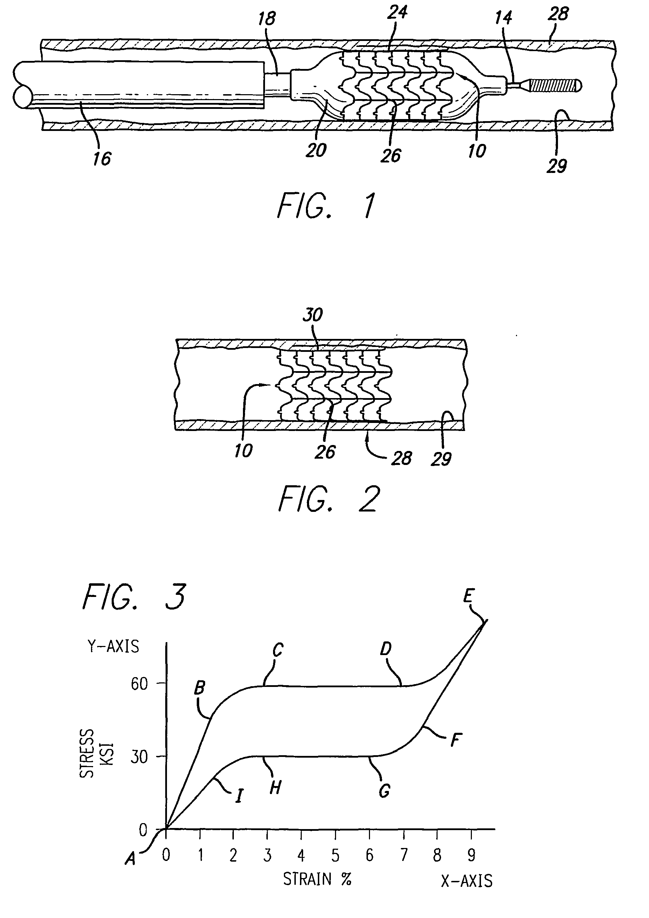

Referring to FIGS. 1, 2, and 4, in a preferred embodiment, a stent 10 of the present invention is formed partially or completely of alloys such as nitinol (NiTi) which have superelastic (SE) characteristics. Stent 10 is somewhat similar to the stent disclosed in United States Patent No. 5,569,295, "Expandable Stents and Method for Making Same," issued to Lam on October 29, 1996. Some differences of the present stent from that disclosed in the '295 patent is that the stent is preferably constructed of a superelastic material with the addition of a ternary element, and the strut pattern has changed. Of course, the configuration of the stent 10 is just one example of many stent configurations that are contemplated.

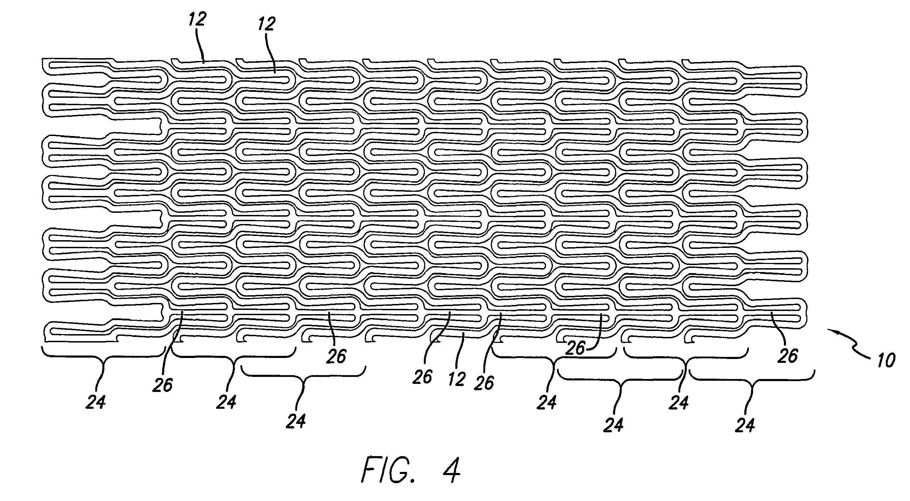

Turning to FIG. 4, stent 10 has a tubular form which preferably includes a plurality of radially expandable cylindrical elements 24 disposed generally coaxially and interconnected by members 26 disposed between adjacent cylindrical elements 24. The shapes of the struts 12 forming the strut pattern are designed so they can preferably be nested. This strut pattern is best seen from the flattened plan view of FIG. 4. The serpentine patterned struts 12 are nested such that the extended portions of the struts of one cylindrical element 24 intrude into a complementary space within the circumference of an adjacent cylindrical element. In this manner, the plurality of cylindrical elements 24 can be more tightly packed lengthwise.

As introduced above, an exemplary stent includes a superelastic material. In a general sense, superelasticity implies that the material can undergo a large degree of reversible strain as compared to common steel. In a technical sense, the term "superelasticity" and sometimes "pseudoelasticity" refer to an isothermal transformation in nitinol. More specifically, it refers to stress inducing a mal-tensitic phase from an austenitic phase. Alloys having superelastic properties generally have at least two phases: a martensitic phase, which has a relatively low tensile strength and which is stable at relatively low temperatures, and an austenitic phase, which has a relatively high tensile strength and which is stable at temperatures higher than the martensitic phase. Superelastic characteristics generally allow the metal stent to be deformed by collapsing the stent and creating stress which causes the NiTi to reversibly change to the martensitic phase. The stent is restrained in the deformed condition inside a delivery sheath typically to facilitate the insertion into a patient's body, with such deformation causing the isothermal phase transformation. Once within the body lumen, the restraint on the stent is removed, thereby reducing the stress thereon so that the superelastic stent returns towards its original undeformed shape through isothermal transformation back to the austenitic phase. Under these conditions, the stent can be described as self-expanding.

Returning to FIG. 1, the graphic illustrates, in a partial cross-sectional view, the distal end of a rapid exchange stent delivery system that includes a guide wire 14, a delivery sheath 16, and an intravascular catheter 18. For the sake of clarity, the illustration of the delivery system in FIG. 1 has been simplified. It is just one example of a delivery system that may be used with the present invention. More details of a delivery system specifically for use with a self-expanding stent may be found in, for example, U.S. Patent No. 6,077,295 to Limon et al., entitled "Self Expanding Stent Delivery System,". Other delivery systems such as over-the-wire may be used without departing from the scope of the instant invention.

FIG. 1 further shows an optional expandable balloon 20 inflated through an inflation lumen (not shown), although the balloon is typically not needed for a self-expanding stent. The stent 10 is first crimped on to the deflated balloon 20, and the entire assembly is kept underneath the delivery sheath 16 until the moment the stent 10 is deployed. The stent 10 is self-expanding so that when the sheath 16 is withdrawn, the stent 10 expands to its larger deployment diameter without assistance from the balloon 20. Nevertheless, some procedures specifically use the balloon 20 to further expand the stent 10 for improved seating in the artery wall 29.

FIG. 2 illustrates the self-expanding stent 10 in the expanded condition after the delivery system has been removed. If an external force is applied to the artery 28, the expanded stent 10 temporarily and at least partially collapses or deforms. As the stent 10 deforms, stress in the nickel-titanium alloy causes an isothermal phase transformation from the austenitic phase to the martensitic phase. When the external force is removed, the stress in stent 10 is likewise diminished so that the stent quickly transforms back from the martensitic phase to the austenitic phase. As this almost instantaneous, isothermal transformation occurs, the stent 10 returns to its fully expanded state and the artery remains open. When the superelastic stent 10 is implanted in an artery 28, its high resilience effectively maintains the patency of the artery while minimizing the risk of permanent arterial collapse at the implant site if the stent is temporarily deformed due to external forces. Furthermore, the resilience of the stent 10 supports the flap 30 to maintain patency of the artery.

Stent 10 is preferably formed from a superelastic material such as nickel-titanium and undergoes an isothermal transformation when stressed if in the austenitic phase. For most purposes, the transformation temperature for the stent 10 is preferably set low enough such that the nickel-titanium alloy is in the austenitic phase while at body temperature.

According to theory, when stress is applied to a specimen of a metal such as nitinol exhibiting superelastic characteristics at a temperature at or above that which the transformation of the martensitic phase to the austenitic phase is complete, the specimen deforms elastically until it reaches a particular stress level where the alloy then undergoes a stress-induced phase transformation from the austenitic phase to the martensitic phase. As the phase transformation progresses, the alloy undergoes significant increases in strain with little or no corresponding increases in stress. The strain increases while the stress remains essentially constant until the transformation of the austenitic phase to the martensitic phase is complete. Thereafter, further increase in stress is necessary to cause further deformation. The martensitic metal first yields elastically upon the application of additional stress and then plastically with permanent residual deformation.

If the load on the specimen is removed before any permanent deformation has occurred, the stress-induced martensite elastically recovers and transforms back to the austenitic phase. The reduction in stress first causes a decrease in strain. As stress reduction reaches the level at which the martensitic phase begins to transform back into the austenitic phase, the stress level in the specimen remains essentially constant (but less than the constant stress level at which the austenitic crystalline structure transforms to the martensitic crystalline structure until the transformation back to the austenitic phase is complete); i.e., there is significant recovery in strain with only negligible corresponding stress reduction. After the transformation back to austenite is complete, further stress reduction results in elastic strain reduction. This ability to incur significant strain at relatively constant stress upon the application of a load and to recover from the deformation upon the removal of the load is commonly referred to as "superelasticity" and sometimes "pseudoelasticity."

FIG. 3 illustrates an idealized shess-strain hysteresis curve for a superelastic, binary nickel-titanium alloy. The relationship is plotted on x-y axes, with the x axis representing strain and the y axis representing stress. For ease of illustration, the x-y axes are labeled on a scale typical for superelastic nitinol, with stress from 0 to 60 ksi and strain from 0 to 9 percent, respectively.

Looking at the plot in FIG. 3, the line from point A to point B represents the elastic deformation of the nickel-titanium alloy. After point B the strain or deformation is no longer proportional to the applied stress and it is in the region between point B and point C that the stress-induced transformation of the austenitic phase to the martensitic phase begins to occur.

At point C moving toward point D, the material enters a region of relatively constant stress with significant deformation or strain. This constant or plateau region is known as the loading stress, since it represents the behavior of the material as it encounters continuous increasing strain. It is in this plateau region C-D that the transformation from austenite to martensite occurs.

At point D the transformation to the martensitic phase due to the application of stress to the specimen is substantially complete. Beyond point D the martensitic phase begins to deform, elastically at first, but, beyond point E, the deformation is plastic or permanent.

When the stress applied to the superelastic metal is removed, the material behavior follows the curve from point E to point F. Within the E to F region, the martensite recovers its original shape, provided that there was no permanent deformation to the martensitic structure. At point F in the recovery process, the metal begins to transform from the stress-induced, unstable, martensitic phase back to the more stable austenitic phase.

In the region from point G to point H, which is also an essentially constant or plateau stress region, the phase transformation from martensite back to austenite takes place. This constant or plateau region G-H is known as the unloading stress. The line from point I to the starting point A represents the elastic recovery of the metal to its original shape.

Binary nickel-titanium alloys that exhibit superelasticity have an unusual stress-strain relationship as just described and as plotted in the curve of FIG. 3. As emphasized above, the superelastic curve is characterized by regions of nearly constant stress upon loading, identified above as loading plateau stress C-D and unloading plateau stress G-H. Naturally, the loading plateau stress C-D always has a greater magnitude than the unloading plateau stress G-H. The loading plateau stress represents the period during which martensite is being stress-induced in favor of the original austenitic crystalline structure. As the load is removed, the stress-induced martensite transforms back into austenite along the unloading plateau stress part of the curve. The difference in stress between the stress at loading C-D and unloading stress G-H defines the hysteresis of the system.

The present invention seeks to preserve the superelastic qualities of nickel-titanium alloys just described yet improve upon the matenial's radiopacity by addition of a ternary element. This is preferably accomplished in one embodiment by forming a composition consisting essentially of about 30 to about 52 percent titanium and the balance nickel and up to 10 percent of one or more additional ternary alloying elements. Such ternary alloying elements may be selected from the group consisting of iridium, platinum, gold, rhenium, tungsten, palladium, rhodium, tantalum, silver, ruthenium, or hafnium. In the preferred embodiment, the atomic percentage of platinum is greater than or equal to 2.5 and less than or equal to 15. In an alternative embodiment, the atomic percentage of palladium is greater than or equal to 2.5 and less than or equal to 20.

A preferred embodiment stent has 42.8 atomic percent nickel, 49.7 atomic percent titanium, and 7.5 atomic percent platinum. Through empirical studies, the aforementioned compositions produce stent patterns having a radiopacity comparable to the same size and pattern stent made from 316 L stainless steel with a 2.7 to 6.5 ÿm gold coating.

In various alternative embodiments, the present invention contemplates the minor addition of a quaternary element, for example, iron, to further enhance the alloy's formability or its thermomechanical properties. The presence of impurities such as carbon or oxygen or the like in the present invention alloy is also possible.

A preferred method of fabricating the superelastic, radiopaque metallic stent entails first fashioning nickel-titanium tubing. The tubing is made from vacuum induction melting nickel and titanium with the ternary element according to the compositions suggested above. The ingot is then remelted for consistency. The ingot is next hot rolled into bar stock, then straightened and sized, and hot or cold formed into a cylinder. The cylinder is gun drilled to form the tubing. Instead of gun drilling, other methods of material removal known in the art may be used, including electric discharge machining (EDM), laser beam machining, and the like. Next, the tubing is cold drawn and annealed repeatedly to achieve the finished dimensions.

Any of the foregoing preferred embodiment steps may be repeated, taken out of sequence, or omitted as necessary depending on desired results. From here on, the tubing follows conventional stent fabrication techniques such as laser cutting the strut pattern, heat setting, etc.

The following are additional processing guide posts to achieve a sufficiently radiopaque stent yet maintaining the superelastic stress-strain behavior of the alloy. Empirical evidence suggests that, in various preferred embodiments, a Ni-Ti-Pd or Ni-Ti-Pt ingot should have the following austenite finish temperature: 0 degrees C ÿ Af ÿ 40 degrees C. The Ni-Ti-Pd or Ni-Ti-Pt tubing should exhibit an austenite finish temperature of: -15 degrees C ÿ Af ÿ 15 degrees C. In an exemplary embodiment, the final laser cut Ni-Ti-Pd or Ni-Ti-Pt stent should exhibit an austenite finish temperature of: 0 degrees C ÿ Af ÿ 37 degrees C. Of course, the Af of the finished laser cut stent can be set as needed by various heat treating processes known in the art.

It is understood that the austenite finish temperature (Af) is defined to mean the temperature at which the material completely reverts to austenite. In technical terms, the Af (and other transformation temperatures As, Ms, Mf) as it applies to an ingot made of Ni-Ti-Pd or Ni-Ti-Pt, for example, is determined by a Differential Scanning Calorimeter (DSC) test, known in the art. The DSC test method to determine transformation temperatures for the ingot is guided by ASTM standard no. F 2004-00, entitled "Standard Test Method For Transformation Temperature Of Nickel-Titanium Alloys By Thermal Analysis."

The "active Af" for the tubing and the finished stent is determined by a bend and free recovery test, also known in the art. In such a test, the tubing is cooled to under the Mf temperature, deformed, and warmed up. While monitoring the increasing temperature, the point of final recovery of the deformation in the tubing approximates the Af of the material. The active Af testing technique is guided by a second ASTM standard entitled "Standard Test Method For Determination Of Transformation Temperature Of Nickel-Titanium Shape Memory Alloys By Bend And Free Recovery," or by equivalent test methods known in the art.

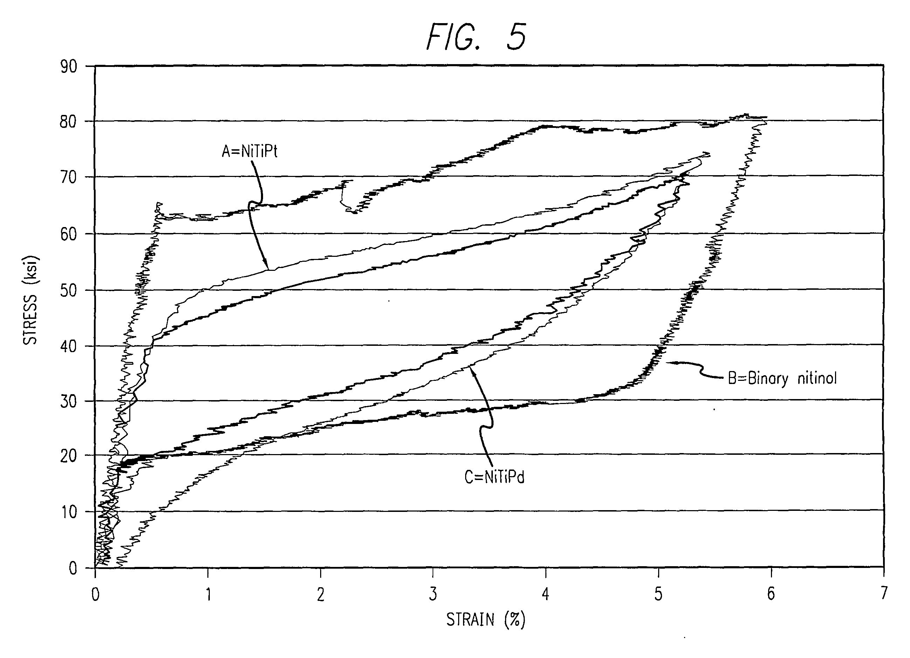

Samples of wire made in accordance with the foregoing exemplary embodiments were tested. Specifically, the stress-strain relationship based on empirical data for nickel-titanium-palladium and nickel-titanium-platinum are plotted against binary nitinol in FIG. 5. Curve A corresponds to a sample of nickel-titanium-platinum. Curve B is based on a sample of binary nitinol. Curve C is based on a sample of nickel-titanium-palladium. To generate the empirical data, the wire samples were placed under increasing tension until past the phase transformation from their initial austenitic phase to their martensitic phase. Tension was then slowly released prior to any plastic deformation until stress on the samples dropped to zero with full deformation recovery.

As is apparent from the plot of FIG. 5, the present invention nickel-titanium-palladium and nickel-titanium-platinum alloys have stress-strain curves that closely follow the hysteresis curve for binary nitinol. All three curves have essentially flat loading and unloading plateau stresses indicating the presence of a phase Transformation that is characteristic of superelastic metals. Hence, the present nitinol stent incorporates a ternary element, in these exemplary embodiments palladium or platinum, to improve radiopacity yet the materials' superelastic capability is preserved. What has been missing heretofor is empirical evidence that this level of radiopacity can be achieved while preserving the superelastic characteristics of these alloys.

The present example further provides a nitinol stent having improved radiopacity without reliance on increasing the stent wall thickness or strut thickness. Increasing wall or strut thicknesses detracts from the flexibility of the stent, which is detrimental to deliverability. Rather, the present superelastic nitinol stent has a thin wall/strut thickness and/or strut cross-sectional area akin to a conventional stainless steel stent, and has comparable radiopacity to a stainless steel stent with a thin coating of gold. The wall/strut thickness is defined by the difference between the inside diameter and the outside diameter of the tube.

Indeed, the improved radiopacity of the present stent can be characterized strictly by strut thickness. In this context, the present radiopaque stent has a reduced strut thickness yet exhibits the radiopacity of an identical stent having thicker struts. In other words, given a stent exhibiting a certain level of radiopacity, the present stent having the identical dimensions and strut pattern achieves that level of radiopacity yet it has at least a 10 percent reduction in strut thickness as compared to the reference stent.

Alternatively, the 10 percent reduction can also be quantified in terms of the cross-sectional area of the strut. That is, for a given stent having a certain level of radiopacity with struts with a given cross-sectional area, the present stent having the same dimensions and strut pattern achieves the same level of radiopacity but has struts with at least a 10 percent reduction in cross-sectional area as compared to the reference stent.

Another aspect of nitinol aside from its superelasticity is shape memory. The present invention can also be employed with respect to this physical attribute as described below.

The shape memory effect allows a nitinol structure to be deformed to facilitate its insertion into a body lumen or cavity, and then heated within the body so that the structure returns to its original, set shape. Nitinol alloys having shape memory effect generally have at least two phases: a martensitic phase, which has a relatively low tensile strength and which is stable at relatively low temperatures, and an austenitic phase, which has a relatively high tensile strength and which is stable at temperatures higher than the martensitic phase.

Shape memory effect is imparted to the alloy by heating the nickel-titanium metal to a temperature above which the transformation from the martensitic phase to the austenitic phase is complete; i.e., a temperature above which the austenitic phase is stable. The shape of the metal during this heat treatment is the shape "remembered." The heat-treated metal is cooled to a temperature at which the martensitic phase is stable, causing the austenitic phase to transform to the martensitic phase. The metal in the martensitic phase is then plastically deformed, e.g., to facilitate the entry thereof into a patient's body. Subsequent heating of the deformed martensitic phase to a temperature above the martensite to austenite transformation temperature causes the deformed martensitic phase to transform to the austenitic phase. During this phase transformation the metal reverts back towards its original shape.

The recovery or transition temperature may be altered by making minor variations in the composition of the metal and in processing the material. In developing the correct composition, biological temperature compatibility must be determined in order to select the correct transition temperature. In other words, when the stent is heated, it must not be so hot that it is incompatible with the surrounding body tissue. Other shape memory materials may also be utilized, such as, but not limited to, irradiated memory polymers such as autocrosslinkable high density polyethylene (HDPEX). Shape memory alloys are known in the art and are discussed in, for example, "Shape Memory Alloys," Scientific American, Vol. 281, pp. 74-82 (November 1979).

Shape memory alloys undergo a transition between an austenitic phase and a martensitic phase at certain temperatures. When they are deformed while in the martensitic phase, they retain this deformation as long as they remain in the same phase, but revert to their original configuration when they are heated to a transition temperature, at which time they transform to their austenitic phase. The temperatures at which these transitions occur are affected by the nature of the alloy and the condition of the material. Nickel-titanium-based alloys (NiTi), wherein the transition temperature is slightly lower than body temperature, are preferred for the present invention. It is desirable to have the transition temperature set at just below body temperature to insure a rapid transition from the martinsitic state to the austenitic state when the stent is implanted in a body lumen.

Turning again to FIGS. 1, 2, and 4, the present stent 10 is formed from a shape memory alloy, such as NiTi discussed above. After the stent 10 is inserted into an artery 28 or other vessel, the delivery sheath 16 is withdrawn exposing the stent 10 to the ambient environment. The stent 10 then immediately expands due to contact with the higher temperature within artery 28 as described for devices made from shape memory alloys. An optional expandable balloon 20 may be inflated by conventional means to further expand the stent 10 radially outward.

Again, if an external force is exerted on the artery, the stent 10 temporarily at least partially collapses. But the stent 10 then quickly regains its former expanded shape due to its shape memory qualities. Thus, a crush-resistant stent, having shape memory characteristics, is implanted in a vessel. It maintains the patency of a vessel while minimizing both the risk of permanent vessel collapse and the risk of dislodgment of the stent from the implant site if the stent is temporarily deformed due to external forces.

When the stent 10 is made in accordance with the present example, it is also highly radiopaque. The same alloying processes described earlier are used here to add the ternary element to increase the radiopacity of the stent. Insofar as the martensitic to austenitic phase transformation is thermally driven, the deployment of the present stent can be explained in terms of the shape memory effect.

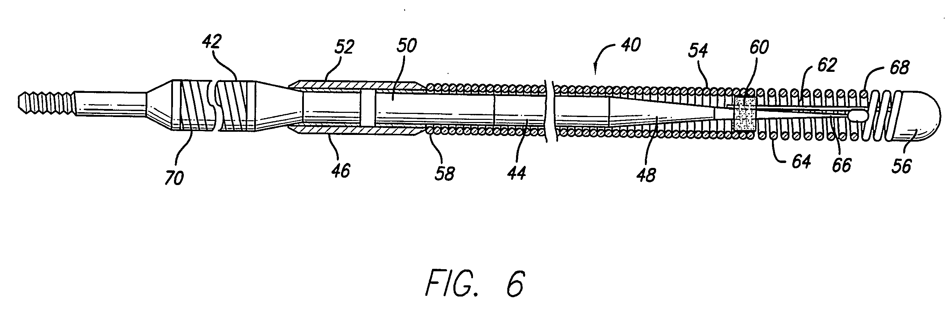

The present invention is applicable to guide wires. FIG. 6 is a side elevational view partially in section of an exemplary embodiment guide wire 40 employing features of the present invention. The guide wire 40 comprises an elongated, relatively high strength proximal portion 42, a relatively short distal portion 44 which is formed substantially of a superelastic alloy material and a connector element 46 formed substantially of a superelastic alloy material and which connects the proximal end of the distal portion 44 to the distal end of the proximal portion in a torque transmitting relationship. The distal portion 44 has at least one tapered section that becomes smaller in the distal direction. The connector element 46 is preferably a hollow, tubular shaped structure having an inner lumen extending therethrough which is adapted to receive the proximal end 50 of the distal portion 44 and the distal end 52 of the proximal portion 42. The ends 50, 52 may be press fit into the connector element 46, or they may be secured therein by crimping, swaging the connector, or by means such as a suitable adhesive, weld, braze, or solder.

A helical coil 54 is disposed about the distal portion 44 and has a rounded plug 56 at the distal end thereof. The helical coil 54 is preferably secured to the distal portion 44 at proximal location 58 and at intermediate location 60 by solder and by the distal end thereof to the rounded plug 56, which is usually formed by soldering or welding the distal end of the helical coil 54 to the distal tip of the optional shaping ribbon 62. Preferably, the most distal section 64 of the helical coil 54 is made of a radiopaque metal such as platinum, or platinum-nickel alloys to facilitate the identification thereof while it is disposed within a patient's body under a fluoroscope or x-ray. The most distal section 64 is preferably stretched about 10 % to about 30 % of the un-stretched length of the helical coil 54.

A most distal part 66 of the distal portion 44 is optionally flattened into a rectangular or square shaped cross-section and preferably provided with a rounded tip 68. The rounded tip 68 may be a bead of solder used to minimize the inadvertent passage of the most distal part 66 through the spacing between the stretched distal section 64 of the helical coil 54.

The exposed portion of the elongated proximal portion 42 may be covered with a coating of lubricious material such as polytetrafluroroethylene (sold under the trademark TEFLON) or other suitable lubricious coatings such as polysiloxane.

The elongated proximal portion 42 of the guide wire 40 is generally about 130 to about 140 cm in length with an outer diameter of about 0,15 mm (0.006 inch) to about 0,45 mm (0.018 inch) for coronary use. Large diameter guide wires may be employed in peripheral arteries and other body lumens. The lengths of the smaller diameter and tapered sections can range from about 2 to about 20 cm, depending upon the stiffness or flexibility desired in the final product. The helical coil 54 is about 20 to about 45 cm in length, has an outer diameter about the same size as the diameter of the elongated proximal portion 42, and is made from wire about 0,05 to 0,08 mm (0.002 to 0.003 inch) in diameter. More than one helical coil can be used. The shaping ribbon and the flattened most distal part 66 of the distal portion 44 may have rectangular cross-sections which usually have dimensions of about 0,025 to 0,08 mm (0.001 by 0.003 inch).

The present invention guide wire 40 includes a superelastic nitinol distal portion 44 and optionally the connector element 46. Both are preferably made in accordance with the nickel-titanium and ternary element compositions described above. The connector element 46 may be a binary nitinol in one alternative embodiment.

A presently preferred method for making the final configuration of the superelastic portions of the guide wire 40 is to cold work, preferably by drawing, a rod or tubular member having a composition according to the relative proportions described above, and then heat treating the cold worked part while it is under stress to impart a shape memory thereto. Typical initial transverse dimensions of the rod or the tubular member are about 1,14 mm (0.045 inch) and about 6,3 mm (0.25 inch) respectively. If the final product is to be tubular, a small diameter ingot, e.g. 6,3 mm to about 38 mm (0.25 to about 1.5 inch) in diameter and 125 to about 760 mm (5 to about 30 inches) in length, may be formed into a hollow tube by extruding or by machining a longitudinal center hole therethrough and grinding the outer surface thereof smooth. Before drawing the solid rod or tubular member, it is optionally annealed at a temperature of about 500ÿC to about 750ÿC, typically about 650ÿC, for about 30 minutes in a protective atmosphere such as argon to relieve essentially all internal stresses. In this manner, all of the specimens start the subsequent thermal mechanical processing in essentially the same metallurgical condition so that products with consistent final properties are obtained. Such treatment also provides the requisite ductility for effective cold working.

The stress-relieved stock is cold worked by drawing to effect a reduction in the cross-sectional area thereof of about 30 % to 70%. The metal is drawn through one or more dies or appropriate inner diameter with a reduction per pass of about 10 % to 50 %. Other forms of cold working can be employed such as swaging.

Following cold work, the drawn wire or hollow tubular product is heat treated at a temperature between 350ÿC and about 600ÿC for about 0.5 to about 60 minutes. Preferably, the drawn wire or hollow tubular product is simultaneously subjected to a longitudinal stress between about 5 % and about 50 %, preferably about 10 % to about 30 % of the tensile strength of the material (as measured at room temperature) in order to impart a straight memory shape to the metal and to insure that any residual stresses therein are uniform. This memory-imparting heat treatment also fixes the austenite-martensite transformation temperature for the cold worked metal. By developing a straight memory and maintaining uniform residual stresses in the superelastic material, there is little or no tendency for a guide wire made of this material to whip when it is torqued within a patient's blood vessel. Other processing details for guide wires can be found in, for example, U.S. Patent No. 5,695,111 to Nanis, et al., or U.S. Patent No. 5,341,818 to Abrams, et al., whose contents are hereby incorporated by reference.

The nitinol hypotube from which the connector element 46 is formed generally may have an outer diameter from about 0,15 mm to about 0,05 mm (0.006 inch to about 0.002 inch) with wall thicknesses of about 0,025 to 0,1 mm (0.001 to about 0.004 inch). A presently preferred superelastic hypotube for the connector element 46 has an outer diameter of about 0.014 inch and a wall thickness of about 0,05 mm (0.002 inch).

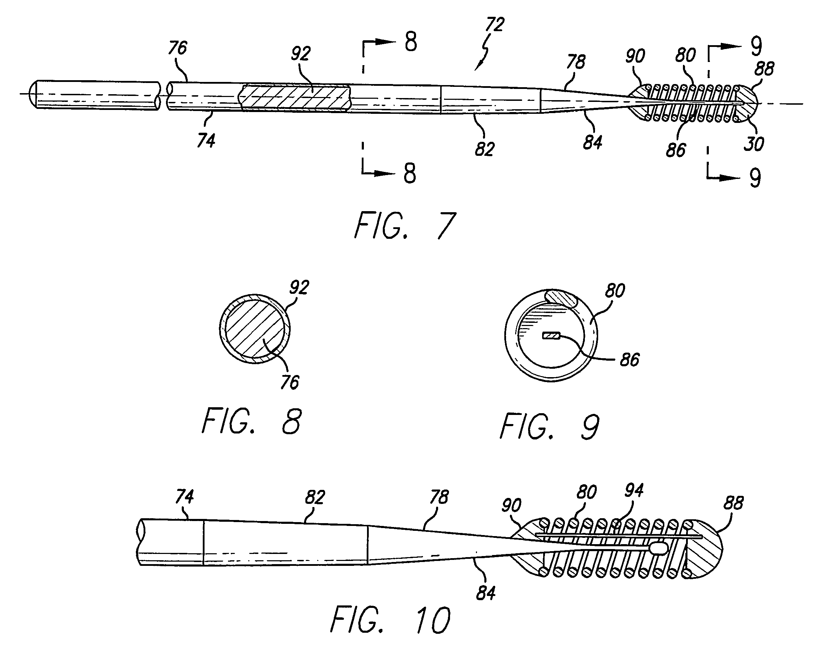

FIG. 7 is a side elevational view partially in section of an alternative embodiment guide wire 72. The guide wire 72 of this exemplary embodiment has a core member 74 with a proximal core section 76, a distal core section 78, and a helical coil 80. The distal core section 78 has a first tapered segment 82 and a second tapered core segment 84 which is distally contiguous to the first tapered core segment 82. The second tapered segment 84 tapers at a greater degree than the first tapered segment 82 and this additional taper provides a much smoother transition when the distal portion of the guide wire 72 is advanced through a tortuous passageway of a patient.

A flattened distal tip 86 extends from the distal end of the second tapered core segment 84 to the body of solder 88 that secures the flattened distal tip 86 of the core member 74 to the distal end of the helical coil 80. A body of solder 90 secures the proximal end of the helical coil 80 to an intermediate location on the second tapered segment 84. As seen in the embodiment of FIG. 7, the core member 74 optionally extends, continuously and uninterrupted, from the proximal end to the distal end of the guide wire 72.

The core member 74 is preferably coated with a lubricious coating 92 such as a fluoropolymer, e.g. TEFLON available from DuPont, which extends the length of the proximal core section 76. The distal core section 78 is also covered with a lubricious coating (not shown) such as a MICROGLIDE coating used by Advanced Cardiovascular Systems, Inc. Hydrophilic coatings may also be employed.

FIG. 10 is a partial side elevational view of the distal core section of an alternative embodiment guidewire that has a separate shaping ribbon extending from the distal extremity of the core member 74 to the distal end of the helical coil 80. In this alternative embodiment, the flattened distal tip 86 of the core member 74 shown in FIG. 7 is replaced with a shaping ribbon 94 which is secured by its distal end to the distal end of the coil 80 and by its proximal end to the distal extremity of the core member 74.

In the embodiments illustrated in FIG. 7 and FIG. 10, the guide wires may employ the superelastic nickel-titanium alloys with a ternary element as described above. It is also possible to use non-superelastic metals such as steels for the core member. For example, the entire core member of the guide wire may be made from high tensile stainless steel, or high-ten 304 stainless steel. Other high strength metals, some of which are precipitation hardenable, include 304 stainless steel, MP35N, and L605.

The tapered segments 82, 84 shown in FIGS. 7 and 10 may be fashioned to create a linear change in stiffness. This is disclosed in, for example, U.S. Patent No. 6,390,993 to Cornish, et al.

## Claims

1. A guide wire (40, 72) for use in a body lumen, comprising:
an elongate core (74) having proximal (42, 76) and distal (44, 78) core sections;
wherein the distal core section (44, 78) includes a superelastic alloy, and **characterised in that** the alloy further includes a ternary element selected from the group of chemical elements consisting of iridium, platinum, gold, rhenium, tungsten, rhodium, tantalum, silver, ruthenium, or hafnium; and
wherein the distal core section (44, 78) is substantially radiopaque.

2. The guide wire of claim 1, wherein the atomic percent of platinum is greater than or equal to 2.5 and less than or equal to 15.

3. The guide wire of claim 1, wherein the substantially radiopaque distal core section (44, 78) is higher in radiopacity than the radiopacity of a similarly dimensioned wire core made from binary nickel-titanium.

4. The guide wire of claim 1, wherein the alloy comprises about 30 to about 52 percent titanium and the balance nickel and up to about 10 percent of one or more additional ternary alloying elements.

5. The guide wire of claim 1, wherein the superelastic alloy includes a nickel-titanium alloy.

6. The guide wire of claim 5, wherein the atomic percent of titanium is greater than or equal to about 49.6 and less than or equal to about 49.8.

7. The guide wire of claim 5, wherein an austenite finish temperature (Af) of the superelastic alloy is greater than or equal to zero and less than or equal to about 37 degrees C.

8. The guide wire of claim 5, wherein the tubular-shaped body includes raw tubing having an austenite finish temperature (Af) of greater than or equal to about -15 degrees C and less than or equal to about 15 degrees C.

9. The guide wire of claim 1, wherein the superelastic alloy is a superelastic nickel-titanium alloy, and wherein the guide wire further comprises a polymer coating (92) at least partially covering the distal core section (44, 78).

10. The guide wire of claim 9, wherein the radiopacity of the distal core section (44, 78) is greater than a radiopacity of a similarly dimensioned distal core section made from binary nickel-titanium.

11. The guide wire of claim 9, wherein the atomic percent of platinum is greater than or equal to about 2.5 and less than or equal to about 15.

12. The guide wire of claim 9, wherein the distal core section (78) and the proximal core section (76) are a single, continuous, uninterrupted length of the alloy.

13. The guide wire of claim 9, wherein the guide wire (40) includes a tubular torque transmitting element (46) joining the distal core section (44) to a discrete proximal core section (42), and the torque transmitting element (46) includes the alloy with a ternary element.

14. A method for providing a guide wire (40, 72) for medical applications, comprising :
providing an elongated core (74) having proximal (42, 76) and distal (44, 78) core sections, wherein the distal core section (44, 78) includes a superelastic nickel-titanium alloy and the alloy further includes a ternary element selected from the group of chemical elements consisting of iridium, platinum, gold, rhenium, tungsten, rhodium, tantalum, silver, ruthenium, or hafnium;
cold drawing the elongated core (74);
heat treating the elongated core (74); and
wherein the distal core section (44, 78) is radiopaque.

15. The method of claim 14, wherein the step of providing an elongated core (74) includes melting nickel, titanium, and the ternary element, cooling to form an alloy ingot, hot forming the alloy ingot, forming the alloy ingot into a cylinder, drawing the cylinder, and annealing the cylinder.

16. The method of claim 14, wherein the atomic percent of platinum is greater than or equal to about 2.5 and less than or equal to about 15,

17. The method of claim 14, wherein an austenite finish temperature (Af) of the superelastic alloy is greater than or equal to about zero and less than or equal to about 37 degrees C.

18. The method of claim 15, wherein the ingot after melting includes an austenite finish temperature (Af) of greater than or equal to about 0 degrees C and less than or equal to about 40 degrees C.

19. The method of claim 15, wherein the tubing includes an austenite finish temperature (Af) of greater than or equal to about 15 degrees C and less than or equal to about 15 degrees C.

20. The method of claim 15, wherein the ingot is remelted.

21. The method of claim 14, wherein the alloy includes a quaternary element.

## Patentansprüche

1. Führungsdraht (40, 72) zur Verwendung in einem Körperlumen, umfassend:
einen länglichen Kern (74), der proximale (42, 76) und distale (44, 78) Kernabschnitte aufweist;
wobei der distale Kernabschnitt (44, 78) eine superelastische Legierung umfasst, und **dadurch gekennzeichnet, dass** die Legierung weiterhin ein ternäres Element umfasst, das aus der Gruppe der chemischen Elemente, die aus Iridium, Platin, Gold, Rhenium, Wolfram, Rhodium, Tantal, Silber, Ruthenium oder Hafnium besteht, ausgewählt ist; und
wobei der distale Kernabschnitt (44, 78) im Wesentlichen strahlenundurchlässig ist.

2. Führungsdraht nach Anspruch 1, wobei die Atomprozente von Platin größer als oder gleich 2,5 und kleiner als oder gleich 15 sind.

3. Führungsdraht nach Anspruch 1, wobei der im Wesentlichen strahlenundurchlässe distale Kernabschnitt (44, 78) eine höhere Strahlenundurchlässigkeit aufweist, als die Strahlenundurchlässigkeit eines ähnlich dimensionierten Führungsdrats aus binärem Nickel-Titan.

4. Führungsdraht nach Anspruch 1, wobei die Legierung etwa 30 bis etwa 52 Prozent Titan und im Übrigen Nickel und bis zu etwa 10 Prozent eines zusätzlichen ternären Legierungselements oder mehrerer zusätzlicher ternärer Legierungselemente umfasst.

5. Führungsdraht nach Anspruch 1, wobei die superelastische Legierung eine Nickel-Titan-Legierung umfasst.

6. Führungsdraht nach Anspruch 5, wobei die Atomprozente von Titan größer als oder gleich etwa 49,6 und kleiner als oder gleich etwa 49,8 sind.

7. Führungsdraht nach Anspruch 5, wobei eine Austenit-Endtemperatur (Af) der superelastischen Legierung größer als oder gleich null und kleiner als oder gleich etwa 37 Grad C ist.

8. Führungsdraht nach Anspruch 5, wobei der rohrförmige Körper Roh-Rohrmaterial umfasst, das eine Austenit-Endtemperatur (Af) größer als oder gleich etwa -15 Grad C und kleiner als oder gleich etwa 15 Grad C aufweist.

9. Führungsdraht nach Anspruch 1, wobei die superelastische Legierung eine superelastische Nickel-Titan-Legierung ist, und wobei der Führungsdraht weiterhin eine Polymerbeschichtung (92) umfasst, die den distalen Kernabschnitt (44, 78) zumindest teilweise bedeckt.

10. Führungsdraht nach Anspruch 9, wobei die Strahlenundurchlässigkeit des distalen Kernabschnitts (44, 78) größer als eine Strahlenundurchlässigkeit eines ähnlich dimensionierten distalen Kernabschnitts aus binärem Nickel-Titan ist.

11. Führungsdraht nach Anspruch 9, wobei die Atomprozente von Platin größer als oder gleich etwa 2,5 und kleiner als oder gleich etwa 15 sind.

12. Führungsdraht nach Anspruch 9, wobei der distale Kernabschnitt (78) und der proximale Kernabschnitt (76) eine einzige, kontinuierliche, ununterbrochene Länge der Legierung sind.

13. Führungsdraht nach Anspruch 9, wobei der Führungsdrat (40) ein rohrförmiges Element zur Übertragung eines Drehmoments (46) umfasst, das den distalen Kernabschnitt (44) mit einem getrennten proximalen Kernabschnitt (42) verbindet, und wobei das Element zur Übertragung eines Drehmoments (46) die Legierung mit einem ternären Element umfasst

14. Verfahren zur Bereitstellung eines Führungsdrats (40, 72) für medizinische Anwendungen, umfassend:
Bereitstellen eines länglichen Kerns (74), der proximale (42, 76) und distale (44, 78) Kernabschnitte aufweist; wobei der distale Kernabschnitt (44, 78) eine superelastische Nickel-Titan-Legierung umfasst, und die Legierung weiterhin ein ternäres Element umfasst, das aus der Gruppe der chemischen Elemente, die aus Iridium, Platin, Gold, Rhenium, Wolfram, Rhodium, Tantal, Silber, Ruthenium oder Hafnium besteht, ausgewählt ist;
Kaltziehen des länglichen Kerns (74);
Wärmebehandeln des länglichen Kerns (74); und
wobei der distale Kernabschnitt (44, 78) strahlenundurchlässig ist.

15. Verfahren nach Anspruch 14, wobei der Schritt Bereitstellen eines länglichen Kerns (74) das Schmelzen von Nickel, Titan und dem ternären Element, das Abkühlen, um einen Legierungsblock zu formen, das Warmformen des Legierungsblocks, wodurch der Legierungsblock zu einem Zylinder geformt wird, das Ziehen des Zylinders und das Glühen des Zylinders umfasst.

16. Verfahren nach Anspruch 14, wobei die Atomprozente von Platin größer als oder gleich etwa 2,5 und kleiner als oder gleich etwa 15 sind.

17. Verfahren nach Anspruch 14, wobei eine Austenit-Endtemperatur (Af) der superelastischen Legierung größer als oder gleich etwa null und kleiner als oder gleich etwa 37 Grad C ist.

18. Verfahren nach Anspruch 15, wobei der Block nach dem Schmelzen eine Austenit-Endtemperatur (Af) größer als oder gleich etwa 0 Grad C und kleiner als oder gleich etwa 40 Grad C umfasst.

19. Verfahren nach Anspruch 15, wobei das Rohrmaterial eine Austenit-Endtemperatur (Af) größer als oder gleich etwa -15 Grad C und kleiner als oder gleich etwa 15 Grad C umfasst.

20. Verfahren nach Anspruch 15, wobei der Block umgeschmolzen wird.

21. Verfahren nach Anspruch 14, wobei die Legierung ein quartäres Element umfasst.

## Revendications

1. Fil de guidage (40, 72) destiné à être utilisé dans une lumière corporelle, comprenant :
un élément central allongé (74) ayant des sections d'élément central proximale (42, 76) et distale (44, 78) ;
dans lequel la section d'élément central distale (44, 78) comprend un alliage super-élastique et **caractérisé en ce que** l'alliage comprend en outre un élément ternaire choisi dans le groupe d'éléments chimiques consistant en l'iridium, le platine, l'or, le rhénium, le tungstène, le rhodium, le tantale, l'argent, le ruthénium et l'hafnium ; et
dans lequel la section d'élément central distale (44, 78) est substantiellement radio-opaque.

2. Fil de guidage suivant la revendication 1, dans lequel le pourcentage atomique de platine est supérieur ou égal à 2,5 et inférieur ou égal à 15.

3. Fil de guidage suivant la revendication 1, dans lequel la section d'élément central distale substantiellement radio-opaque (44, 78) a une radio-opacité supérieure à la radio-opacité d'un élément central de fil ayant des dimensions similaires produit à partir de nickel-titane binaire.

4. Fil de guidage suivant la revendication 1, dans lequel l'alliage comprend environ 30 à environ 52 % de titane et le pourcentage restant de nickel et jusqu'à environ 10 % d'un ou plusieurs éléments ternaires supplémentaires d'alliage.

5. Fil de guidage suivant la revendication 1, dans lequel l'alliage super-élastique comprend un alliage nickel-titane.

6. Fil de guidage suivant la revendication 5, dans lequel le pourcentage atomique de titane est supérieur ou égal à environ 49,6 et inférieur ou égal à environ 49,8.

7. Fil de guidage suivant la revendication 5, dans lequel une température de finition d'austénite (Af) de l'alliage super-élastique est supérieure ou égale à zéro et inférieure ou égale à environ 37°C.

8. Fil de guidage suivant la revendication 5, dans lequel le corps de forme tubulaire comprend une tubulure brute ayant une température de finition d'austénite (Af) supérieur ou égale à environ -15°C et inférieure ou égale à environ 15°C.

9. Fil de guidage suivant la revendication 1, dans lequel l'alliage super-élastique est un alliage nickel-titane super-élastique, et ledit fil de guidage comprenant en outre un revêtement polymère (92) couvrant au moins partiellement la section d'élément central distale (44, 78).

10. Fil de guidage suivant la revendication 9, dans lequel la radio-opacité de la section d'élément central distale (44, 78) est supérieure à la radio-opacité d'une section d'élément central distale de dimensions similaires produite à partir de nickel-titane binaire.

11. Fil de guidage suivant la revendication 9, dans lequel le pourcentage atomique de platine est supérieur ou égal à environ 2,5 et inférieur ou égal à environ 15.

12. Fil de guidage suivant la revendication 9, dans lequel la section d'élément central distale (78) et la section d'élément central proximale (76) sont constituées d'une longueur unique, continue, ininterrompue de l'alliage.

13. Fil de guidage suivant la revendication 9, ledit fil de guidage (40) comprenant un élément de transmission de moment de torsion tubulaire (46) joignant la section d'élément central distale (44) à une section d'élément central proximale discrète (42), et l'élément de transmission de moment de torsion (46) comprend l'alliage avec un élément ternaire.

14. Procédé pour fournir un fil de guidage (40, 72) pour des applications médicales, comprenant les étapes consistant à :
fournir un élément central allongé (74) ayant des sections d'élément central proximale (42, 76) et distale (44, 78), la section d'élément central distale (44, 78) comprenant un alliage nickel-titane super-élastique et l'alliage comprenant en outre un élément ternaire choisi dans le groupe d'éléments chimiques consistant en l'iridium, le platine, l'or, le rhénium, le tungstène, le rhodium, le tantale, l'argent, le ruthénium et l'hafnium ;
étirer à froid l'élément central allongé (74) ;
soumettre à un traitement thermique l'élément central allongé (74) ; et
dans lequel la section d'élément central distale (44, 78) est radio-opaque.

15. Procédé suivant la revendication 14, dans lequel l'étape de fourniture d'un élément central allongé (74) comprend la fusion de nickel, de titane et de l'élément ternaire, un refroidissement pour former un lingot d'alliage, un façonnage à chaud du lingot d'alliage, la transformation du lingot d'alliage en un cylindre, l'étirage du cylindre et le recuit du cylindre.

16. Procédé suivant la revendication 14, dans lequel le pourcentage atomique de platine est supérieur ou égal à environ 2,5 et inférieur ou égal à environ 15.

17. Procédé suivant la revendication 14, dans lequel une température de finition d'austénite (Af) de l'alliage super-élastique est supérieure ou égale à environ zéro et inférieure ou égale à environ 37°C.

18. Procédé suivant la revendication 15, dans lequel le lingot, après fusion, comprend une température de finition d'austénite (Af) supérieure ou égale à environ 0°C et inférieure ou égale à environ 40°C.

19. Procédé suivant la revendication 15, dans lequel la tubulure a une température de finition d'austénite (Af) supérieure ou égale à environ -15°C et inférieure ou égale à environ 15°C.

20. Procédé suivant la revendication 15, dans lequel le lingot est soumis à une refonte.

21. Procédé suivant la revendication 14, dans lequel l'alliage comprend un élément quaternaire.
